# EUROPÄISCHE PATENTANMELDUNG

(11) **EP 0 564 859 A1**
(43) Veröffentlichungstag der Anmeldung: **13.10.1993**
(21) Anmeldenummer: 93104384.8
(22) Anmeldetag: 17.03.1993
(51) Int. Cl.: A61B 17/34, A61M 25/01

(54) **Anästhesiebesteck**

(30) Priorität: 09.04.1992 DE 9204919 U
(71) Anmelder: B. Braun Melsungen AG, D-34209 Melsungen (DE)
(72) Erfinder: Bain, Mike, W-6600 Saarbrücken (DE); Wiegel, Heinz, W-6445 Alheim (DE)
(74) Vertreter: Selting, Günther, Dipl.-Ing.

(57) **Zusammenfassung**

Ein Anästhesiebesteck, bestehend aus einer Epiduralkanüle (111) mit abgebogener Spitze (112) und einem Kanülenansatz (115) und aus einer Spinalkanüle (30) mit einem Kanülenansatz (33), die mit ihrem vorderen Ende durch ein axiales Loch (114) der Epiduralkanüle (111) vorschiebbar ist, zeichnet sich dadurch aus, daß der Kanülenansatz (115) der Epiduralkanüle (111) eine radiale Nase (40) aufweist und an dem anderen Kanülenansatz (33) eine axial gerade verlaufende Führungsspur (121) für die radiale Nase (40) ausgebildet ist und daß sich an das innere Ende der Führungsspur (121) ein umfangsmäßiger Sperrbereich (122) anschließt, mit dem die Nase (40) bei Relativdrehung der beiden Kanülenansätze (115) zu ihrer gegenseitigen Verriegelung zusammenwirkt.

Auf diese Weise wird die Durchführung einer kombinierten Spinal-/Epiduralanästhesie für den Anwender erleichtert und für den Patienten sicherer.

## Beschreibung

Die Erfindung bezieht sich auf ein Anästhesiebesteck, bestehend aus einer Epiduralkanüle mit abgebogener Spitze und aufwärts gerichteter Schlifföffnung, bei der in der äußeren Wand der Krümmung der Spitze ein Loch ausgebildet ist, und aus einer spitz angeschliffenen Spinalkanüle, die länger und dünner als die Epiduralkanüle ist und die mit ihrem vorderen Ende durch das Loch der Epiduralkanüle vorschiebbar ist, wobei am hinteren Ende jeder Kanüle ein Kanülenansatz befestigt ist und beide Kanülenansätze konzentrische Paßteile aufweisen, die axial zusammensteckbar sind.

Bei Operationen der unteren Extremitäten und der Beckenorgane sowie in der Geburtshilfe stehen zur Regionalanästhesie im wesentlichen die Spinalanästhesie und die Epiduralanästhesie zur Verfügung. Vorteile der Spinalanästhesie sind schneller Wirkungseintritt, kaum Versager und meist vollständige Anästhesie bzw. Analgesie. Die Nachteile bestehen darin, daß sie eingeschränkt in der Höhe wirksam ist, daß die Wirkdauer bei einmaliger Injektion (single shot) begrenzt ist. Bei der Epiduralanästhesie wird durch eine Epiduralkanüle ein Katheter in den Epiduralraum eingebracht. Beliebige Anästhesiedauer durch Nachspritzen und postoperative Analgesierung sind erreichbar.

Bei der kombinierten Spinal-/Epiduralanästhesie wird zuerst die Epiduralkanüle in üblicher Technik im Epiduralraum plaziert und dann wird die Spinalkanüle durch die Epiduralkanüle hindurch bis in den Spinalraum vorgeschoben. Nach der Spinalanästhesie wird die Spinalkanüle entfernt und ein Epiduralkatheter mittels bereits liegender Epiduralkanüle plaziert.

Die Vorteile dieser Technik sind folgende:
1. Zur Durchführung der beiden unterschiedlichen Anästhesien ist nur eine Punktion erforderlich;
2. sofortiger Wirkungseintritt und Operationsbeginn;
3. geringe Medikamentendosis vorab bedingt durch die Spinal-Blockade;
4. intra-operative Verlängerung durch den liegenden Epidural-Katheter möglich;
5. Sicherung der post-operativen Analgesierung;
6. keine Handhabungsschwierigkeiten mit der dünnen Spinalnadel (Epidural-Nadel fungiert als Einführkanüle);
7. Bestätigung beider Punktionsarten durch Liquorrückfluß bzw. Loss of Resistance-Methode;
8. über den Epidural-Katheter kann durch Titration des Medikamentes ein höherer Block erreicht werden.

Zur Durchführung der kombinierten Spinal-/Epiduralanästhesie sind unterschiedliche Anästhesiebestecks entwickelt worden. Kanülensets für eine solche Technik bestehen aus einer Epiduralkanüle von 88 mm (3½") Länge mit abgebogener Spitze und aufwärts gerichteter Öffnung und einer längeren Spinalkanüle, deren äußerer Durchmesser kleiner als der Innendurchmesser der Epiduralkanüle ist.

Bei einem bekannten derartigen Kanülenset (DE-A-33 27 585) wird durch eine Epiduralkanüle mit abgebogener Spitze und seitwärts gerichteter Schlifföffnung eine Spinalkanüle mit Mandrin hindurchgeschoben, bis ihr vorderes Ende durch die aufwärts gerichtete Schlifföffnung der plazierten Epiduralkanüle hindurchtritt und die Dura durchsticht. Das Durchdringen der Dura ist hierbei für den Anwender nicht oder kaum spürbar, weil die gerade Spinalkanüle in der Biegung der Epiduralkanüle klemmt und mit Kraftaufwand vorgeschoben werden muß. Um dabei die senkrecht verlaufenden Fasern der Dura möglichst wenig zu beschädigen, ist die Schlifföffnung der Spinalkanüle zur etwa waagerechten Schlifföffnung der Epiduralkanüle um etwa 90° gedreht und diese gegenseitige Orientierung wird durch Zusammengriff eines Vorsprunges an dem Ansatz der einen Kanüle mit einem axialgerichteten Schlitz an dem Ansatz der anderen Kanüle gesichert. Nach dem Entfernen des Mandrins und erfolgtem Liquoraustritt wird die Anästhesie durch die Spinalkanüle eingeleitet. Zu diesem Zweck wird an ihren Kanülenansatz eine Spritze angesetzt. Nach Herausziehen der Spinalkanüle aus der Epiduralkanüle wird durch die Epiduralkanüle ein Epiduralkatheter eingeführt. Um dabei zu vermeiden, daß der Epiduralkatheter durch das von der Spinalkanüle in die Dura gestochene Loch in den Spinalraum eintritt, wird empfohlen, die Epiduralkanüle nach dem Entfernen der Spinalkanüle um 180° zu drehen, so daß ihre Schlifföffnung in entgegengesetzte Richtung weist und der Epiduralkatheter von dem Loch in der Dura weggelenkt wird.

Bei einem anderen Anästhesiebesteck, das aus DE-A-39 22 406 bekannt ist und dessen Merkmale im Oberbegriff des Anspruches 1 aufgeführt sind, wird die Epiduralkanüle mit aufwärts in Richtung Kopf des Patienten weisender Schlifföffnung in den Epiduralraum gebracht, damit der Epiduralkatheter zur Erzielung optimaler Anästhesiewirkung kopfwärts vordringt. Die Spinalkanüle wird auf der Längsachse der Epiduralkanüle zentriert vorgeschoben, bis sie durch das in der äußeren Wand der Krümmung der Spitze ausgebildete koaxiale Loch hindurchtritt und in die Dura eindringt. Wegen der Leichtgängigkeit des Vorschubes der Spinalkanüle kann die Punktion der Dura vom Anästhesisten sehr gut wahrgenommen werden. Es ist zweckmäßig, wenn die Ebene der Schlifföffnung der Spinalkanüle bei der Punktion der Dura senkrecht orientiert ist, damit nur wenige der senkrecht verlaufenden Durafasern bei der Punktion zerschnitten werden und wenn nach Durchdringung der Dura die Schlifföffnung der Spinalkanüle entsprechend der Schlifföffnung der Epiduralkanüle nach oben zeigt, damit das Injektionsmittel sich in dem Spinalraum nach oben verteilen kann. Bei diesem Anästhesiebesteck braucht die Epiduralkanüle nach der Spinalanästhesie nicht gedreht zu werden, weil das Loch in der Dura jenseits der Schlifföffnung der Epiduralkanüle liegt, und zwar - wenn die Schlifföffnung ordnungsgemäß nach oben gerichtet ist - sich unterhalb der Schlifföffnung befindet. Allerdings hat auch dieses Anästhesiebesteck einen Mangel. Die Kanülenansätze der Epiduralkanüle und der Spinalkanüle sind nicht aufeinander abgestimmt. Bei der Spinalanästhesie wird die Spinalkanüle nur von der Dura gehalten, so daß ein unbeabsichtigtes Verschieben und Verdrehen der Spinalkanüle im Spinalraum beim Aufsetzen der Spritze und der Injektion sehr leicht möglich ist. Zwar läßt sich bei den beiden bekannten Anästhesiebestecken die Spinalkanüle in der Epiduralkanüle drehen und im Falle DE-A-33 27 585 in der Einstich-Winkelstellung sichern, jedoch kann der Anwender die relativen Schliffstellungen von Epidural- und Spinalkanüle nicht in jeder Phase der kombinierten Spinal-/Epiduralanästhesie auf einfache Weise gezielt einstellen und sichern.

Aus DE-A-32 18 242 ist bei einer Doppelkanüle ausschließlich für die Epidural-Anästhesie bekannt, eine Innenkanüle mit geschlossenem stumpfen Ende und seitlicher Öffnung in einer äußeren Stichkanüle mit axialer Öffnung in zwei axialen Stellungen festzulegen. Zu diesem Zweck hat die Innenkanüle am hinteren Ende radial abstehende Stifte, die entweder in Anschlagkerben am hinteren Ende der Stichkanüle axial lose anliegen oder in zu diesen umfangsmäßig versetzte Bajonett-Schlitze mit kurzem Verriegelungsschenkel eingreifen. In der einen Stellung verschließt die stumpfe Spitze der Innenkanüle das Lumen der offenen Spitze der Stichkanüle und in der anderen Stellung ist die seitliche Öffnung der Innenkanüle freigegeben und lenkt den durch ihr Lumen hindurchgeführten Epiduralkatheter aus der Axialrichtung seitwärts ab. Eine für die kombinierte Spinal-/Epiduralanästhesie geeignete vorgegebene Orientierung und Sicherbarkeit von zwei angeschliffenen Kanülen ergibt sich hierbei nicht.

Der Erfindung liegt die Aufgabe zugrunde, ein Anästhesiebesteck für die kombinierte Spinal-/Epiduralanästhesie so zu verbessern, daß die Durchführung einer solchen kombinierten Anästhesie für den Anwender erleichtert und für den Patienten sicherer wird.

Diese Aufgabe wird erfindungsgemäß durch die Merkmale des Anspruches 1 gelöst.

Die Führungsspur an dem Paßteil des einen Kanülenansatzes wirkt mit der radialen Nase des Paßteiles des anderen Kanülenansatzes derart zusammen, daß die Spinalkanüle an der Führungsspur gerade geführt in der Epiduralkanüle vorgeschoben wird und mit vorgegebener Ausrichtung ihrer Schlifföffnung (um 90° zu der kopfwärts gerichteten Schlifföffnung der Epiduralkanüle gedreht) aus dem Loch in der Krümmung der Epiduralkanüle vortritt, um die Dura zu durchtrennen und in den Spinalraum einzudringen (Grundposition). Sobald die Spinalkanüle um das gewünschte Maß, maximal 10 mm, aus der Epiduralkanüle herausragt, wird ihr Kanülenansatz gedreht, so daß die Nase über den umfangsmäßig orientierten Sperrbereich läuft und dabei sowohl eine Veränderung der Orientierung der Schlifföffnung der Spinalkanüle in bezug auf die Schlifföffnung der Epiduralkanüle als auch durch axiale Verriegelung der beiden Kanülenansätze eine Sicherung der Spinalkanüle gegen Verschiebung im Spinalraum erreicht werden (Endposition).

Zweckmäßige Ausgestaltungen der Erfindung sind in den Unteransprüchen angegeben.

Vorteilhafterweise ist gemäß Anspruch 2 die Führungsspur als Abflachung eines kreiszylindrischen Teiles vorgesehen und die Nachbarzonen bilden Sperrbereiche für die Nase. Bevorzugt ist der kreiszylindrische Teil ein Stutzen des Epiduralkanülenansatzes und die Nase befindet sich an der Innenfläche einer den Stutzen überfangenden Kappe, die als Paßteil des Spinalkanülenansatzes dient. Nach 10 mm Weg endet die Abflachung an einer inneren Schulter, gegen die die Nase trifft, so daß die Spinalkanüle maximal 10 mm vor der Vorderkante der Epiduralkanülenspitze stehen kann. Der Anwender kann die Spinalkanüle, sobald er die Dura durchstochen hat, durch eine Drehung nach links oder rechts in der Epiduralkanüle blockieren. Die Blockierung geschieht durch den reibenden Sitz der Nase auf dem jeweiligen Wölbungsbereich des Paßteiles neben der Abflachung. Falls nach dem Arretieren der Liquorfluß versiegt, weil Nervenfasern vor der Schlifföffnung liegen, kann ohne die axiale Arretierung aufzuheben die Spinalkanüle weiter oder in die andere Richtung gedreht werden, um ihre Schlifföffnungsorientierung zu verändern.

Eine weitere günstige Ausgestaltung der Erfindung ist in Anspruch 4 wiedergegeben.

Um dabei zu erreichen, daß die Nase des Paßteiles des einen Kanülenansatzes bereits in den axial gerichteten Schenkel der Nut eingreift, bevor die Spitze der Spinalkanüle durch das Loch in der Krümmung der Epiduralkanüle hindurchtritt, ist vorzugsweise die Nut L-förmig gestaltet und ihr längerer Schenkel verläuft axial. Seine Länge ist abgestimmt auf den Überstand der Spinalkanüle aus der Epiduralkanüle. Die Nase gelangt durch Relativdrehung der Kanülenansätze in eine Richtung in den umfangsmäßigen kürzeren Schenkel der Nut und legt einen 90°-Weg zurück, so daß nun die Schlifföffnung der Spinalkanüle in die gleiche Richtung (aufwärts) zeigt wie die Schlifföffnung der Epiduralkanüle und die Injektion durchgeführt werden kann.

Der Zusammenhalt der Spinalkanüle und der Epiduralkanüle in Injektionsposition der Spinalkanüle wird vorzugsweise dadurch gesichert, daß die Nase an dem Sperrbereich in um 90° zur Grundposition gedrehter Endposition verrastet ist. Zu diesem Zweck kann bei der ersten Ausführungsform der Erfindung eine Abflachung der Wölbung des kreiszylindrischen Teiles dienen und bei der zweiten Ausführungsform kann der Nutengrund mit einer Vertiefung zur Aufnahme der Nase versehen sein.

Um dem Anwender die gegenseitige Ausrichtung der Schlifföffnungen und der Führungsorgane jeweils kenntlich zu machen, ist vorgesehen, daß der Kanülenansatz der Spinalkanüle einen seiner Führungsspur bzw. Nase gegenüberliegenden axialen und/oder äußeren radialen Orientierungsvorsprung aufweist.

Nach der Spinalanästhesie wird bei beiden Ausführungsformen der Erfindung die Spinalkanüle durch eine Drehung entriegelt und aus der Epiduralkanüle herausgezogen. Ein Epiduralkatheter kann sodann wie üblich vorgeschoben werden und es kann über den Epiduralkatheter nachdosiert oder auch eine Schmerztherapie nach einer Operation durchgeführt werden.

In der Zeichnung sind zwei Ausführungsbeispiele der Erfindung schematisch dargestellt. Es zeigt:
Fig. 1 eine Draufsicht auf eine erste Ausführungsform des Kanülenansatzes einer Epiduralkanüle,
Fig. 2 eine Stirnansicht gegen die Anordnung nach Figur 1 - gesehen in Richtung des Pfeiles A -,
Fig. 3 eine perspektivische Ansicht der Epiduralkanüle im Zustand der Einführung einer Spinalkanüle,
Fig. 4 einen Längsschnitt durch die Anordnung nach Figur 3 längs der Linie III-III,
Fig. 5 einen Querschnitt durch den Kanülenansatz der Spinalkanüle längs der Linie V-V in Figur 4,
Fig. 6 einen Querschnitt durch die Anordnung während des Zusammensteckens der Kanülen,
Fig. 6A die Ausrichtung der Schlifföffnungen der beiden Kanülen im Zustand der Zusammensteckung der Figur 6,
Fig. 7 einen Querschnitt durch die Anordnung nach Beendigung des Zusammensteckens mit verriegelten Kanülen,
Fig. 7A die Ausrichtung der Schlifföffnungen der beiden Kanülen im Zustand der Verriegelung gemäß Figur 7,
Fig. 8 eine perspektivische Ansicht einer zweiten Ausführungsform eines Anästhesiebesteckes im Zustand der Einführung der Spinalkanüle in die Epiduralkanüle,
Fign. 9 und 10 zwei aufeinanderfolgende Vorschubstadien der Spinalkanüle,
Fig. 11 einen Schnitt längs der Linie XI-XI in Figur 9,
Fig. 12 einen Schnitt längs der Linie XII-XII in Figur 10,
Fig. 13 die Endposition der um 90° zu der Epiduralkanüle gedrehten Spinalkanüle und
Fig. 14 einen Schnitt durch die Führungs- und Verriegelungsvorrichtung längs der Linie XIV-XIV in Figur 13.

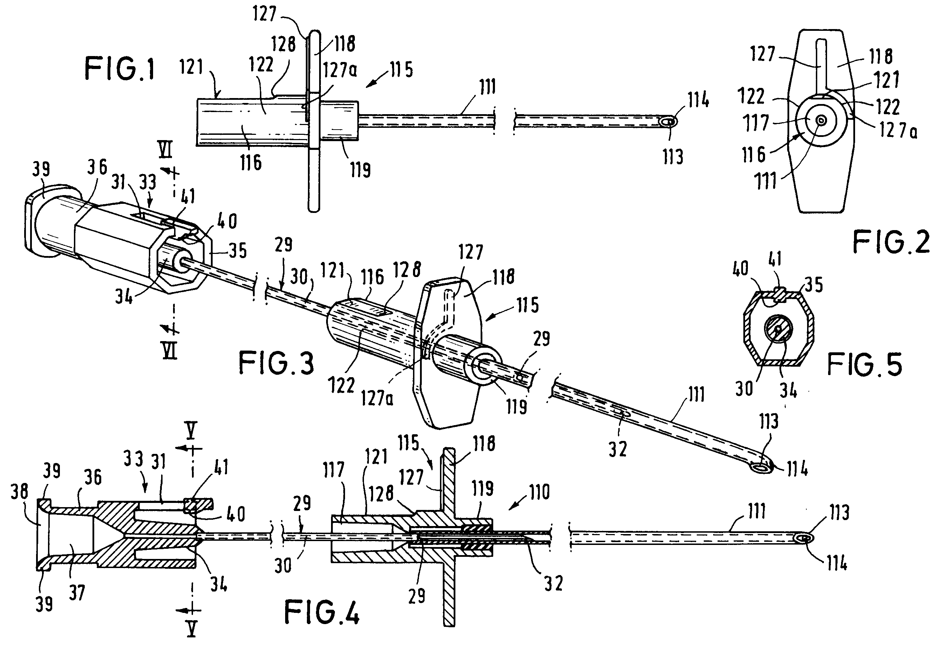

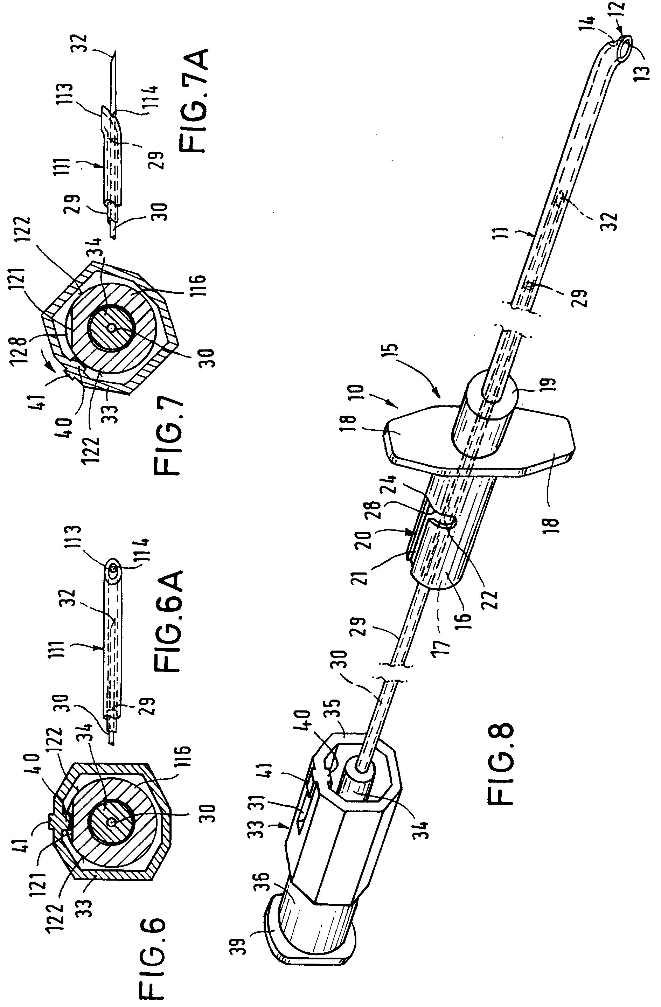

Ein Anästhesiebesteck zur kombinierten Spinal-/Epiduralanästhesie wird bezüglich des allgemeinen Aufbaus anhand der Darstellung der Figur 8 erläutert, die diesbezüglich auch auf das Beispiel der Figuren 1 - 7 zutrifft. Es besteht im wesentlichen aus einer Epiduralkanüle 11 und einer in dieser verschiebbaren dünneren und längeren Spinalkanüle 30. Die Epiduralkanüle 11 ist aus einem geraden Kanülenrohr aus Stahl mit Markierungsringen zur Kontrolle der Einschublänge hergestellt. Die Spitze 12 der Epiduralkanüle 11 ist nach oben abgebogen, so daß eine Krümmung mit einer äußeren Wand und einer inneren Wand entsteht, die eine aufwärts gerichtete Schlifföffnung 13 aufweist. Die Schlifföffnung 13 verläuft im wesentlichen parallel zur zylindrischen Wand der Epiduralkanüle 11 und ist mit einem für die Gewebepunktion geeigneten Schliff versehen. Es handelt sich bei der Epiduralkanüle 11 um eine Kanüle des Tuohy-Typs, in der ein nicht gezeichneter Mandrin, vorzugsweise aus Kunststoff, steckt, der nach der Plazierung der Epiduralkanüle 11 im Epiduralraum herausgezogen wird. In der Außenwand der Krümmung der Spitze 12 befindet sich ein Loch 14, das auf der Längsmittelachse des Kanülenrohres der Epiduralkanüle 11 zentriert ist. Am patientenfernen Ende der Epiduralkanüle 11 ist ein Kanülenansatz 15 befestigt. Der Kanülenansatz 15 besteht aus einem Kunststoffteil, das einen rohrförmigen kreiszylindrischen Stutzen 16 mit konischem Hohlraum 17 aufweist und zwei radial gerichtete Griffplatten 18 trägt. An die der Kanülenspitze zugewandte Seite der Griffplatten 18 ist einstückig ein kreisförmiger Ansatz 19 angeformt, auf den eine Kunststoff-Schutzröhre klemmend aufsteckbar ist.

Die Spinalkanüle 30 ist länger und sehr viel dünner als die Epiduralkanüle 11. Sie besteht aus einem dünnen Kanülenrohr, dessen gerade Spitze bei diesem Beispiel mit einer koaxialen endständigen Schlifföffnung 32 versehen ist. Der scharfe Anschliff kann zur Erzielung minimalen Punktionstraumas als Quincke-Schliff ausgeführt sein. Die Spinalkanüle 30 paßt durch das Loch 14 in der Krümmung der Epiduralkanüle 11, so daß die Spinalkanüle 30 durch die Epiduralkanüle 11 hindurch koaxial aus dem Loch 14 vorgeschoben werden kann. Da die Spinalkanüle 30 wesentlich dünner als der Innendurchmesser der Epiduralkanüle 11 ist, befindet sich ein Adapterschlauch 29 auf einem Stück der Spinalkanüle 30. Dieser Adapterschlauch 29 führt die Spinalkanüle 30 in der Epiduralkanüle 11. Das Teil der Spinalkanüle 30, das aus der Epiduralkanüle 11 ragt bzw. sich in dem Loch 14 befindet, wird nicht von dem Adapterschlauch 29 bedeckt. Der Überstand der Spinalkanüle 30 beträgt im Endzustand in diesem Beispiel maximal 10 mm. Am patientenfernen Ende der Spinalkanüle 30 ist ein Kanülenansatz 33 aus Kunststoff befestigt. Dieser besteht im wesentlichen aus einem die Verbindung mit dem Spinalkanülenende herstellenden Außenkonus 34, der in den Innenkonus 17 des Kanülenansatzes 15 paßt und der mit radialem Abstand von einer Kappe 35 umgeben ist, die den rohrförmigen Stutzen 16 des Kanülenansatzes 15 überfängt. An das hintere Ende der Kappe 35 ist ein Rohrkörper 36 angeformt, der einen Innenkonus 37 zur Aufnahme z.B. eines Spritzenkegels aufweist und an dessen Öffnung 38 zwei entgegengesetzt radial gerichtete Nockenvorsprünge 39 ausgebildet sind, die zur Verriegelung mit einer Kupplung eines Anschlußteiles dienen. Das Lumen der Spinalkanüle 30 wird von einem haarfeinen Metallmandrin ausgefüllt, der sich bis zur Schlifföffnung 32 erstreckt und dessen Ansatzstück mit dem Innenkonus 37 des Kanülenansatzes 33 zusammengreift. Der Kanülenansatz 33 hat auf der die Nase 40 tragenden Oberseite ein Fenster 31, das sich axial erstreckt und das die Anbringung der Nase 40 herstellungstechnisch erleichtert.

Bei dem Beispiel der Figuren 1 bis 7 ist in die Außenfläche des kreiszylindrischen Stutzens 116 des Epiduralkanülenansatzes 115 eine Abflachung 121 eingearbeitet, die auf einer Kreis-Sehnenzone liegt und steigungslos ist. Die Abflachung 121 ist gerade und verläuft in Richtung der Längsachse der Kanüle 111 und des Stutzens 116. Die ebene gerade Abflachung 121 ist bei dem Beispiel etwa 10 mm lang und endet an einer inneren Schulter 128. Der Abflachung 121 umfangsmäßig benachbarte Sperrbereiche 122 sind infolge der Kreiszylinderform des Stutzens 116 gewölbt und in bezug auf die Abflachung 121 erhaben. Ggf. kann zur Verrastung der Nase 40 in Injektionsposition der Spinalkanüle 30 eine nicht gezeichnete Abflachung des Stutzens 16 dienen, die um 90° zur Abflachung 121 versetzt ist.

Am öffnungsseitigen Rand der Kappe 35 des Kanülenansatzes 33 ist auf der Wandinnenfläche eine radial nach innen gerichtete Nase 40 ausgebildet, die so bemessen ist, daß sie mit sehr geringem Spiel auf die Abflachung 121 des Kanülenansatzes 115 der Epiduralkanüle 111 paßt und auf dieser bis zur Schulter 128 axial gleitend verschiebbar ist. Zur besseren Identifizierung der Schlifföffnung 32 der Spinalkanüle 30 steht die Nase 40 über den Rand der Kappe 35 axial hinaus. Während die Abflachung 121 rechtwinklig zu der die Schlifföffnung 113 der Epiduralkanüle 111 durchquerenden Axialebene versetzt ist, liegt die Nase 40 in der durch die Spitze der Schlifföffnung 32 der Spinalkanüle 30 verlaufenden Axialebene. Dies hat zur Folge, daß bei Aufführung der Nase 40 auf die Abflachung 121 die Schlifföffnung 32 der Spinalkanüle 30 zur nach oben weisenden Schlifföffnung 113 der Epiduralkanüle 111 um 90° gedreht ist (Figuren 3,4,6A). Die Spinalkanüle 30 wird in dieser Position durch den Zusammengriff von Abflachung 121 und Nase 40 geradlinig geführt, durch das Loch 14 hindurch in den Spinalraum vorgeschoben, wobei nur wenige der senkrecht verlaufenden Durafasern zerschnitten werden. Zur Durchführung der Spinalanästhesie wird der Mandrin aus der Spinalkanüle 30 herausgezogen. Nach Durchdringung der Dura soll die Schlifföffnung 32 der Spinalkanüle 30 - wie diejenige der Epiduralkanüle 111 - im wesentlichen nach oben zeigen, damit das Injektionsmittel sich nach oben verteilen kann (Fig. 7A). Zu diesem Zweck wird der Kanülenansatz 33 relativ zum Kanülenansatz 115 nach rechts oder links gedreht, so daß die Nase 40 auf dem zylindrischen verdickten Sperrbereich 122 des Stutzens 116 klemmt (Fig. 7). Auf dem der Abflachung 121 zugeordneten Flügel der Griffplatte 118 der Epidural-Kanüle 111 befindet sich eine radiale Erhebung 127, die im Bereich des Stutzens 116 eine umfangsmäßige Verlängerung 127a aufweist, welche in Richtung der Ebene der Schlifföffnung 113 verläuft und dem Arzt die Einführrichtung der Spinalkanüle 30 in die Epiduralkanüle 111 und die vorgesehene Drehrichtung andeutet. Die Punktion der Dura ist für den Anwender gut wahrnehmbar. Bei dem Anschluß einer Spritze an den Kanülenansatz 33 und der Injektion ist ein Verschieben der Spinalkanüle 30 im Spinalraum nicht möglich.

Nach der Spinalanästhesie wird die Spinalkanüle 30 durch eine entgegengesetzte Drehung entriegelt und aus der Epiduralkanüle 111 herausgezogen. Durch diese wird in üblicher Weise ein Epiduralkatheter in den Epiduralraum eingeführt und es wird auch die Epiduralkanüle 111 anschließend abgezogen.

Als weitere Handhabungshilfe für den Arzt dient ein axialer und ggf. zusätzlich radialer Orientierungsvorsprung 41, der auf der Außenfläche der Kappe 35 an gleicher Stelle wie die Nase 40 angebracht ist. Nase 40 und Orientierungsvorsprung 41 liegen in der Flucht des Fensters 31. Der Anwender kann mit Hilfe des Orientierungsvorsprunges 41 die Spinalkanüle 30 in bezug auf die Epiduralkanüle 111 von vornherein korrekt ausrichten, so daß die Nase 40 ohne unnötige Manipulationen direkt auf die Abflachung 121 aufläuft und die Führung der Spinalkanüle 30 mit rechtwinklig zur Schlifföffnung 113 ausgerichteter Schlifföffnung 32 übernimmt.

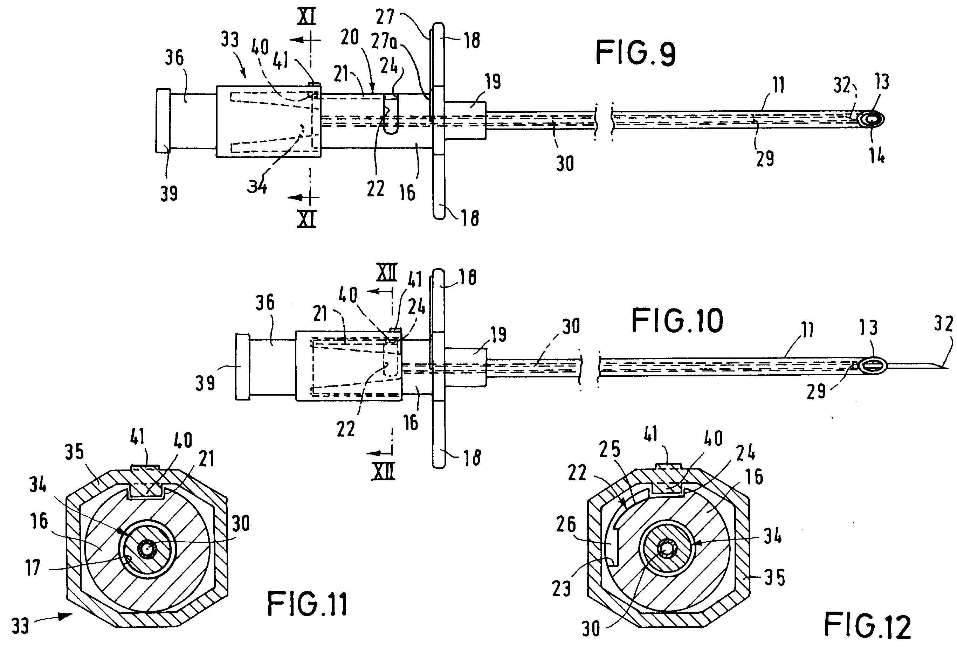

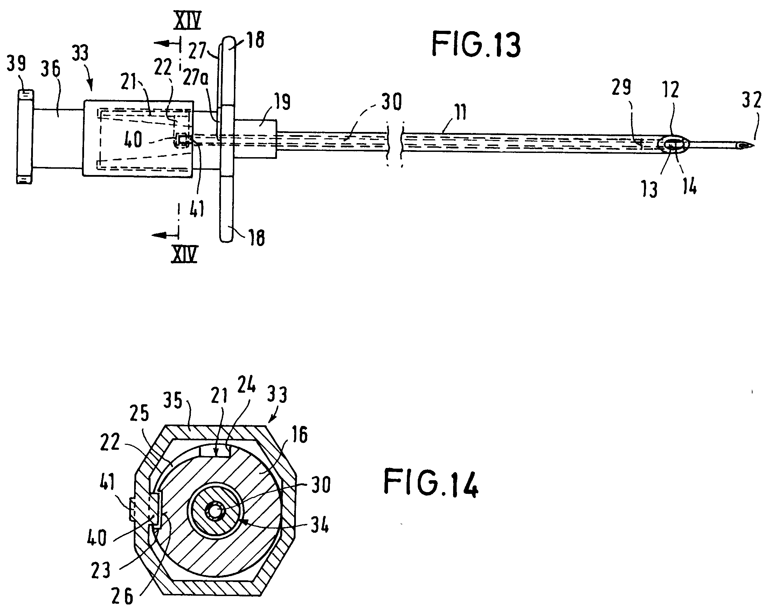

Bei dem Beispiel gemäß Figuren 8 bis 14 ist der Aufbau der Spinalkanülenanordnung mit derjenigen nach Figuren 1 bis 7 identisch. In die Außenfläche des Stutzens 16 des Kanülenansatzes 15 der Epiduralkanüle 11 allerdings ist eine rechtwinklige Nut 20 eingearbeitet, die L-Form hat. Der längere Schenkel 21 der Nut 20 ist gerade und verläuft in Richtung der Längsachse der Kanüle und des Stutzens 16. Er ist nach hinten offen. An das vordere, innere Ende des längeren Schenkels 21 schließt sich rechtwinklig ein kürzerer Schenkel 22 der Nut 20 als Sperrbereich an, der sich über 90° des Umfanges des Stutzens 16 erstreckt und durch eine Endfläche 23 (Figuren 12 und 14) geschlossen ist. Der Grund des kürzeren Schenkels 22 der Nut 20 ist wie in Figuren 12 und 14 erkennbar profiliert. Von dem Scheitel 24 der Nut 20 verläuft er über eine nach außen gerichtete Wölbung 25 zu einer Vertiefung 26, die am Ende des Schenkels 22 von der Endfläche 23 begrenzt ist und die um 90° zum Scheitel 24 versetzt ist. Die Vertiefung 26 dient als Rastelement wie nachfolgend im einzelnen erläutert ist.

Die radial nach innen gerichtete Nase 40 am öffnungsseitigen Rand der Kappe 35 des Spinalkanülenansatzes 33 ist so bemessen, daß sie mit sehr geringem Spiel auf die Abflachung 20 des Kanülenansatzes 15 der Epiduralkanüle 11 paßt und auf dieser gleitend bis zur Schulter 28 verschiebbar ist. Während der axial verlaufende Schenkel 21 der Nut 20 um 90° zu der die Schlifföffnung 13 der Epiduralkanüle 11 durchquerenden Axialebene versetzt ist, liegt die Nase 40 in der durch die Spitze der Schlifföffnung 32 der Spinalkanüle 30 verlaufenden Axialebene. Dies hat zur Folge, daß bei Einführung der Nase 40 in den Schenkel 21 der Nut 20 die Ebene der Schlifföffnung 32 der Spinalkanüle 30 zur Ebene der Schlifföffnung 13 der Epiduralkanüle 11 rechtwinklig gerichtet ist (Figuren 8 bis 10). Die Spinalkanüle 30 wird in dieser Position durch den Nut/Nase-Zusammengriff geradlinig geführt, durch das Loch 14 hindurch in den Spinalraum vorgeschoben, wobei nur wenige der senkrecht verlaufenden Durafasern zerschnitten werden. Zur Durchführung der Spinalanästhesie wird der Mandrin aus der Spinalkanüle 30 herausgezogen. Nach Durchdringung der Dura soll die Schlifföffnung 32 der Spinalkanüle 30 - wie diejenige der Epiduralkanüle 11 - nach oben zeigen, damit das Injektionsmittel sich nach oben verteilen kann. Zu diesem Zweck wird der Kanülenansatz 33 relativ zum Kanülenansatz 15 nach rechts gedreht, so daß die Nase 40 in den umfangsmäßigen Schenkel 22 der Nut 20 einläuft, die Kurvenkrümmung 25 passiert und bei Erreichen der Vertiefung 26 (Fig. 14) eine verrastete Endposition einnimmt, in der die Schlifföffnung 32 um 90° gedreht ist und die in Figur 13 erkennbare Lage einnimmt, die der Lage der Schlifföffnung 13 der Epiduralkanüle 11 entspricht. Die Punktion der Dura ist für den Anwender gut wahrnehmbar. Bei dem Anschluß einer Spritze an den Kanülenansatz 33 und der Injektion ist ein Verschieben der Spinalkanüle 30 im Spinalraum nicht möglich.

Nach der Spinalanästhesie wird die Spinalkanüle 30 durch eine 90°-Linksdrehung entriegelt und aus der Epiduralkanüle 11 herausgezogen. Durch diese wird in üblicher Weise ein Epiduralkatheter in den Epiduralraum eingeführt und es wird auch die Epiduralkanüle 11 anschließend abgezogen.

Als Handhabungshilfe für den Arzt dient der axiale und radiale Orientierungsvorsprung 41, der auf der Außenfläche der Kappe 35 an gleicher Stelle wie die Nase 40 angebracht ist und dessen Aufgabe im Zusammenhang mit der Beschreibung des Beispieles der Figuren 1 bis 7 erläutert ist.

## Patentansprüche

1. Anästhesiebesteck, bestehend aus einer Epiduralkanüle (111) mit abgebogener Spitze (112) und aufwärts gerichteter Schlifföffnung (113), bei der in der äußeren Wand der Krümmung der Spitze (112) ein Loch (114) ausgebildet ist und aus einer spitz angeschliffenen Spinalkanüle (30), die länger und dünner als die Epiduralkanüle (111) ist und die mit ihrem vorderen Ende durch das Loch (114) der Epiduralkanüle (111) vorschiebbar ist,
wobei am hinteren Ende jeder Kanüle (111;30) ein Kanülenansatz (115;33) befestigt ist und beide Kanülenansätze (115;33) konzentrische Paßteile aufweisen, die axial zusammensteckbar sind,
**dadurch gekennzeichnet**, daß der Paßteil (116 bzw. 35;16 bzw. 35) des einen Kanülenansatzes (115 bzw. 33;15 bzw. 33) eine radiale Nase (40) aufweist und an dem Paßteil (116 bzw. 35;16 bzw. 35) des anderen Kanülenansatzes (33 bzw. 115;33 bzw. 15) eine axial gerade verlaufende Führungsspur (121;21) für die radiale Nase (40) ausgebildet ist, wobei die axiale Führungsspur (121;21) bzw. die Nase (40) in bezug auf die ihrem Kanülenansatz zugehörige Spinalkanüle (30) in der durch die Spitze der Schlifföffnung (32) der Spinalkanüle (30) verlaufenden Axialebene liegt und die axiale Führungsspur (121;21) bzw. die Nase (40) in bezug auf die ihrem Kanülenansatz zugehörige Epiduralkanüle (111;11) rechtwinklig zu der ihre Schlifföffnung (113;13) durchquerenden Axialebene versetzt ist und daß sich an das innere Ende der Führungsspur (121;21) ein umfangsmäßiger Sperrbereich (122;22) anschließt, mit dem die Nase (40) bei Relativdrehung der beiden Kanülenansätze (115 bzw. 33;15 bzw. 33) zu ihrer gegenseitigen Verriegelung zusammenwirkt.

2. Anästhesiebesteck nach Anspruch 1,
**dadurch gekennzeichnet**, daß das Paßteil (116 bzw. 35) des einen Kanülenansatzes (115 bzw. 33) kreiszylindrisch gestaltet ist und an einer axialen Kreis-Sehnenzone eine die Führungsspur bildende Abflachung (121) aufweist und daß die der Abflachung (121) benachbarten Zonen des Paßteiles (116 bzw. 35) Sperrbereiche (122) bilden.

3. Anästhesiebesteck nach Anspruch 2,
**dadurch gekennzeichnet**, daß die Abflachung (121) an einer inneren Schulter (128) endet.

4. Anästhesiebesteck nach Anspruch 1,
**dadurch gekennzeichnet**, daß an dem Paßteil (16 bzw. 35) des einen Kanülenansatzes (15 bzw. 33) eine rechtwinklige Nut (20) ausgebildet ist, deren einer am hinteren Ende offener axialer Schenkel (21) die Führungsspur bildet und deren anderer, den Sperrbereich bildender Schenkel (22) sich über einen 90°-Bereich umfangsmäßig erstreckt und daß die radiale Nase (40) des Paßteiles (35 bzw. 16) des anderen Kanülenansatzes (33 bzw. 15) in der Nut (20) geführt verschiebbar ist.

5. Anästhesiebesteck nach Anspruch 4,
**dadurch gekennzeichnet**, daß die Nut (20) L-förmig gestaltet und ihr längerer Schenkel (21) axial gerichtet ist.

6. Anästhesiebesteck nach einem der Ansprüche 1 bis 5,
**dadurch gekennzeichnet**, daß die Nase (40) an dem Sperrbereich (122;22) in um 90° zur Grundposition gedrehter Endposition verrastet ist.

7. Anästhesiebesteck nach einem der Ansprüche 1 bis 6,
**dadurch gekennzeichnet**, daß der Kanülenansatz (33) der Spinalkanüle (30) einen seiner Führungsspur (121;21) bzw. Nase (40) gegenüberliegenden axialen und/oder radialen äußeren Orientierungsvorsprung (41) aufweist.

8. Anästhesiebesteck nach einem der Ansprüche 1 bis 7,
**dadurch gekennzeichnet**, daß am Kanülenansatz (115;15) der Epiduralkanüle (111;11) eine Griffplatte (118;18) angeordnet ist, deren der Führungsspur (121;21) zugeordneter Flügel auf einer Fläche eine radiale Erhebung (127;27) aufweist, die mit einer umfangsmäßigen Verlängerung (127a;27a) versehen ist, die in Richtung der die Schlifföffnung (113;13) der Epiduralkanüle (111;11) durchquerenden Axialebene verläuft.
